(19) Europäisches Patentamt
European Patent Office
Office européen des brevets

(11) EP 2 823 317 B1

(12) **EUROPEAN PATENT SPECIFICATION**

(45) Date of publication and mention
of the grant of the patent:
**03.08.2016 Bulletin 2016/31**

(21) Application number: **13712889.8**

(22) Date of filing: **06.03.2013**

(51) Int Cl.:
***G01N 33/68*** *(2006.01)*      ***G06F 19/00*** *(2011.01)*

(86) International application number:
**PCT/GB2013/050540**

(87) International publication number:
**WO 2013/132245 (12.09.2013 Gazette 2013/37)**

(54) **METHOD FOR CHARACTERISING PLASMA CELL ASSOCIATED DISEASES**

VERFAHREN ZUR CHARAKTERISIERUNG VON PLASMAZELLENASSOZIIERTEN ERKRANKUNGEN

PROCÉDÉ DE CARACTÉRISATION DE MALADIES ASSOCIÉES AUX CELLULES DE PLASMA

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.03.2012 GB 201203938**

(43) Date of publication of application:
**14.01.2015 Bulletin 2015/03**

(73) Proprietor: **The Binding Site Group Limited Birmingham, West Midlands B15 1QT (GB)**

(72) Inventors:
• **HARDING, Stephen**
**Birmingham**
**West Midlands B15 1QT (GB)**
• **HUGHES, Richard**
**Birmingham**
**West Midlands B15 1QT (GB)**
• **CARR-SMITH, Hugh**
**Birmingham**
**West Midlands B15 1QT (GB)**

(74) Representative: **Elsy, David**
**Withers & Rogers LLP**
**4 More London Riverside**
**London SE1 2AU (GB)**

(56) References cited:

• Alison Levoguer: "Freelite update and Hevylite unpublished data : clinical applications for multiple myeloma", , 8 February 2012 (2012-02-08), XP055066267, Retrieved from the Internet: URL:http://szpiczak.org/lang/aktualnosci/s potkania/rok_2012/pdf/relacja_12_02_06/al_ freelite_update_and_hevylite_unpublished_d ata__for_poland_february_2012.pdf [retrieved on 2013-06-11]
• HEINZ LUDWIG ET AL: "Provide Prognostic Information, Allow Creation of a Prognostic Model and Identify Clonal Changes (clonal tiding) Through the Course of Multiple Myeloma (MM).", 53RD ANNUAL MEETING AND EXPOSITION OF THE AMERICAN SOCIETY OF HEMATOLOGY (ASH), 30 December 2011 (2011-12-30), XP055066336,
• HARI PARAMESWARAN ET AL: "2877 Immunoglobulin Free Light Chain (FLC) and Heavy Chain/Light Chain (HLC) Assays - Comparison with Electrophoretic Responses in Multiple Myeloma (MM)", 53RD ANNUAL MEETING AND EXPOSITION OF THE AMERICAN SOCIETY OF HEMATOLOGY (ASH), 30 December 2011 (2011-12-30), XP055066327,
• XAVIER LELEU ET AL: "Novel M-Component Based Biomarkers in Waldenström's Macroglobulinemia", CLINCIAL LYMPHOMA, MYELOMA & LEUKEMIA, vol. 11, no. 1, 1 February 2011 (2011-02-01), pages 164-167, XP055066493, ISSN: 2152-2650, DOI: 10.3816/CLML.2011.n.039

• A LEGG ET AL: "Serum free light chain and Hevylite analyses in the diagnosis, monitoring and prognosis of B cell disorders", KLINICKA BIOCHEMIE A METABOLISMUS, vol. 18, no. 2, 1 January 2010 (2010-01-01), pages 56-61, XP055066507,

**Description**

The invention relates to an improved method of characterising a plasma cell disease in a patient and to apparatus for carrying out the method.

The Applicants have for many years studied free light chains as a way of assaying for a wide-range of monoclonal gammopathies in patients. The use of such free light chains in diagnosis is reviewed in detail in the book "Serum Free Light Chain Analysis, Sixth Edition (2008) A.R. Bradwell, ISBN 9780704427969". Polyclonal abnormalities, where there is decreased or increased production of polyclonal antibodies in patients is also known.

Antibodies comprise heavy chains and light chains. They usually have a two-fold symmetry and are composed of two identical heavy chains and two identical light chains, each containing variable and constant region domains. The variable domains of each light-chain/heavy-chain pair combine to form an antigen-binding site, so that both chains contribute to the antigen-binding specificity of the antibody molecule. Light chains are of two types, κ and λ and any given antibody molecule has either light chain but never both. There are approximately twice as many κ as λ molecules produced in humans, but this is different in some mammals. Usually the light chains are attached to heavy chains. However, some unattached "free light chains" (FLC) are detectable in the serum or urine of individuals. Free light chains may be specifically identified by raising antibodies against the surface of the free light chain that is normally hidden by the binding of the light chain to the heavy chain. In free light chains this surface is exposed, allowing it to be detected immunologically. Commercially available kits for the detection of κ or λ free light chains include, for example, "Freelite™", manufactured by The Binding Site Limited, Birmingham, United Kingdom. The Applicants have previously identified that measuring the amount of free κ, free λ and/or free κ/free λ ratios, allows the detection of monoclonal gammopathies in patients. It has been used, for example, as an aid in the diagnosis of intact immunoglobulin multiple myeloma, (MM), light chain MM, non-secretory MM, AL amyloidosis, light chain deposition disease, smouldering MM, plasmacytoma and MGUS (monoclonal gammopathies of undetermined significance). Detection of FLC has also been used, for example, as an aid to the diagnosis of other B-cell dyscrasia and indeed as an alternative to urinary Bence Jones protein analysis for the diagnosis of monoclonal gammopathies in general.

Conventionally, an increase in one of the λ or κ light chains is looked for. For example, multiple myeloma (MM) results from the monoclonal multiplication of a malignant plasma cell, resulting in an increase in a single type of cell producing a single type of immunoglobulin. This results in an increase in the amount of free light chain, either λ or κ, observed within an individual. This increase in concentration may be determined, and usually the ratio of the free κ to free λ is determined and compared with the normal range. This aids in the diagnosis of monoclonal disease. Moreover, the free light chain assays may also be used for the following of treatment of the disease in patients. Prognosis of, for example, patients after treatment for AL amyloidosis may be carried out.

Katzmann et al (Clin. Chem. (2002); 48(9): 1437-1944) discuss serum reference intervals and diagnostic ranges for free κ and free λ immunoglobulins in the diagnosis of monoclonal gammopathies. Individuals from 21-90 years of age were studied by immunoassay and compared to results obtained by immunofixation to optimise the immunoassay for the detection of monoclonal free light chains in individuals with B-cell dyscrasia.
The amount of κ and λ FLC and the κ/λ ratios were recorded allowing a reference interval to be determined for the detection of B-cell dyscrasias.

The Applicants have also previously identified that assaying for FLC can be used to predict long-term survival of individuals, even when the individual is an apparently healthy subject. (WO 2011/021041). They found that total FLC concentration is statistically, significantly linked to long-term survival. Moreover, this link appears to be similar or better than the link for existing long-term survival prognostic markers such as cholesterol, creatinine, cystatin C and C-reactive protein. Assays for total FLC measurement are disclosed in the document. Assays measuring total FLC are available from The Binding Site, Birmingham, United Kingdom under the trademark "Combylite".

The measurement of heavy chain class to light chain type bound specific (HLC) immunoglobulins such as IgAλ, IgAκ, IgGκ, IgGλ, IgMκ or IgMλ, also has been found to assist in characterising monoclonal gammopathies. Heavy chain class-light chain type specific antibodies and their use are disclosed in WO 2006/079816. Such antibodies are commercially available from The Binding Site, Birmingham, United Kingdom under the trademark "Hevylite".

Diseases associated with polyclonal abnormalities, where more than one specific antibody has increased or decreased production are generally known. For example, this may be characterised by a general increase in antibody production or by two or more monoclonal antibodies, from two separate tumour sources, being present. Chronic infections, autoimmune diseases and many tumours cause increases in polyclonal immunoglobulins. Skin, pulmonary and gut diseases are more likely to cause increases in IgA concentrations, whilst systemic infections will increase all immunoglobulins, but especially IgG.

Serum protein electrophoresis (SPE) involves scanning agarose electrophoretic gels after serum proteins have been separated and the gel has been stained. Immunofixation electrophoresis (IFE) detects the presence of serum proteins using antibodies binding to the proteins within electrophoretic gels to produce visible precipitation bands. There are a number of limitations associated with such methods. Since 2001, serum FLC assays have been available to identify free λ or free κ light chains (FLC) in serum. The combined use of FLC, SPE and/or IFE assays is recommended in international guidelines. The separate results of each test are interpreted separately, though the combined results together may or may not support one another, see for example Dispenzieri A. et. al, Leukaemia 23-2 (2009), 215-24.

More recently the HLC assays using Hevylite™ antibodies have been developed by the Applicants. Such HLC assays allow a quantifiable ratio between involved monoclonal proteins from tumours that can otherwise be measured by SPE and the uninvolved polyclonal levels of the same heavy chain class but bound to the opposite light chain to be measured. This ratio can be used in place of SPE and IFE for the determination of type and concentration of monoclonal proteins in the serum.

The interpretation of results from the various assays that are available can be difficult and require some skill and expertise. The Applicant has identified that the FLC and HLC results, optionally together with the total FLC and/or total HLC can be weighted to produce a score for the indication of monoclonal gammopathies. This can be used, not only to show an indication of the abnormality, but also a degree of confidence of this indication. Analytical errors in one or more of the results can also be more readily identified. This is expected to allow the distinction and degree of confidence in monoclonal gammopathies and the distinction between:

a) Monoclonal protein production (be this intact immunoglobulins, free light chains or indeed production of both). With or without an associated polyclonal suppression of non-involved immunoglobulins.

b) Elevation of polyclonal production with no monoclonal production - hypergammaglobulineamia.

c) Suppression of polyclonal production with no monoclonal production - hypogammaglobulineamia.

d) Normal polyclonal production with no monoclonal production.

e) Production of kappa light chains or lambda light chains bound to a class of heavy chain and/or production of a kappa light chain or lambda light chain without monoclonal protein production.

The invention provides a method for characterising a plasma cell associated disease in a patient comprising:

(i) providing at least one sample from the patient;

(ii) determining in the sample(s) two or more of;

(a) the κ:λ free light chain (FLC) ratio;
(b) the ratio of κ light chains bound to a class of heavy chain : λ light chain bound to the same class of heavy chain (HLC κ : HLC λ ratio);
(c) the total amount of FLC in the sample(s) and
(d) the total amount of κ light chains bound to the heavy chain class plus λ light chains bound to the same heavy chain class (total HLC);

(iii) comparing each ratio or amount from (a), (b), (c) and/or (d) to predetermined values and assigning a score to each amount or ratio; and

(iv) using the scores to characterise the plasma cell associated disease.

An indication of an abnormal FLC κ:λ ratio or HLC κ : HLC λ ratio, elevated clonal concentration and suppression of uninvolved immunoglobulins, for example, gives a high level of certainty of the presence of a monoclonal plasma-cell associated disease.

Typically the κ:λ FLC ratio and the HLCκ:HLCλ ratio are determined.

Determining the total FLC and total HLC in the samples may also be used to provide scores.

Elevated levels of kappa FLC or lambda FLC with FLC κ : λ ratio within the normal range highlights potential abnormality and warrants further investigation. Similarly elevated HLCκ or HLCλ with HLCκ : HLCλ ratio within the normal range also highlights a potential abnormality and warrants further investigation.

The sample may be a urine sample, though serum, blood or plasma is preferred.

The scoring system allows the characterisation of the plasma-cell associated disease and assists in simplifying the characterisation, allowing assays to be carried out by less skilled practitioners and allowing a degree of confidence in the results to be determined.

The plasma cell associated disease may be a monoclonal gammopathy. It may be B-cell associated diseases such as myeloma, (such as intact immunoglobulin myeloma, light chain myeloma, non-secretory myeloma), an MGUS, AL amyloidosis, Waldenström's macroglobulinaemia, Hodgkin's lymphoma, follicular centre cell lymphoma, chronic lymphocytic leukaemia, mantle cell lymphoma, pre-B cell leukaemia or acute lymphoblastic leukaemia.

The plasma cell associated disease may also be a polyclonal associated disease such as hypergammaglobulineamia or hypogammaglobulineamia.

The method may be computer implemented, for example step (iii) may be implemented. The scores may be displayed on an output such as a computer display. Alternatively, the score may be used to produce an output of the likely diagnosis of the type of disease.

One or more samples may be taken from the patient at substantially the same time. The amounts may be assayed on a single sample or separate samples.

The scores may be used to indicate the type of plasma cell disease and/or include a confidence level for the characterisation of the plasma cell disease.

For example, a high or medium FLC $\kappa$:$\lambda$ ratio would indicate a kappa monoclonal gammopathy. However, a lower confidence of monoclonal production would be indicated where a lower abnormal $\kappa$:$\lambda$ FLC ratio was identified on its own. If the HLC ratio for one of the heavy chain classes (such as IgA$\kappa$:IgA$\lambda$) also indicates the abnormal ratio, for the ratio and light chain type, this confirms the finding of monoclonal production.

The summation of the total FLC and/or total HLC for a specific class, can be used to show abnormal levels of immunoglobulin production or for example suppression of one or more classes of immunoglobulins such as IgM or IgA.

Typically the FLC $\kappa$:$\lambda$ ratio is determined and compared to a normal range for FLC $\kappa$:$\lambda$ ratio. The normal range may be the typical range of $\kappa$:$\lambda$ for apparently healthy individuals which, depending on the population being assessed is typically 0.26 - 1.65.

If the FLC $\kappa$:$\lambda$ ratio is above or below that normal range it may be given a score. The score may be, for example, high, medium or low; 1, 2, 3 or 4; or +, ++, +++ or ++++ for the indication of clonality. A "normal ratio" may also be scored as, for example, zero. A ratio of above the predetermined range, such as above 1.65 indicates kappa monoclonality, so the kappa value would be given a score of, for example, high, medium or low; or 4 for very high, 3 for less high, 2 for lower, 1 just above the normal range. Where the ratio is below the normal range (below 0.26 for example) indicates $\lambda$ monoclonality and again can be scored in a similar way.

For example the following ratios may be used to score the results.

Table 1

| FLC ratio | Score | Result |
|---|---|---|
| >50 | high | FLC$\kappa$ high |
| 20-50 | medium | FLC$\kappa$ medium |
| 1.65-20 | low | FLC$\kappa$ low |
| 0.19-0.26 | low | FLC$\lambda$ low |
| 0.1-0.19 | medium | FLC$\lambda$ medium |
| <0.1 | high | FLC$\lambda$ high |

Alternative scoring systems such as 1 to 4 or + to ++++ may also be used.

If the FLC $\kappa$:$\lambda$ ratio is normal, then the total amount of FLC ($\kappa$+$\lambda$) may be determined and scored to indicate increased or decreased polyclonal FLC production. A normal total of FLC is typically 27 mg/ml in serum. Again scores of high, medium, low; -4 to +4 (where +4 indicates very high total FLC and -4 very low production) or ----, ---, --, -, 0 (normal), +, ++, +++, ++++ may be used. For example:

The amount of $\lambda$ FLC in the sample may be compared to the amount of $\kappa$ FLC in the sample (or the amount of HLC $\kappa$ compared to HLC $\lambda$) to produce a clonality score or an indication of the production or suppression of the FLCs or HLCs. Typically the production or suppression levels are indicated for normal FLC or HLC ratios.

Table 2

For Abnormal FLC Ratios

| Amount of FLC (mg/L) | Amount of Other FLC (mg/L) | Clonal Score |
|---|---|---|
| FLC$\kappa$ > 19.4 | $\lambda$ < 5.71 | +++ $\kappa$ |
|  | 5.71 < $\lambda$ < 26.3 | ++K |
|  | $\lambda$ > 26.3 | + FLC |
| FLC $\lambda$ > 26.3 | $\kappa$ < 3.3 | +++ $\lambda$ |

(continued)

| For Abnormal FLC Ratios | | |
|---|---|---|
| Amount of FLC (mg/L) | Amount of Other FLC (mg/L) | Clonal Score |
| | 3.3 < κ < 19.4 | ++ λ |

Normal ratios (e.g. between 0.26 - 1.65) can also be broken down.

For example

FLC κ + FLC λ > 45.7 mg/L - FLC production

FLC κ + FLC λ < 9.01 mg/L - FLC suppression.

Table 3

| Total FLC (mg/L serum) | Score | Result |
|---|---|---|
| >100 | high | High polyclonal production |
| 50-100 | medium | Medium polyclonal production |
| 27-50 | low | Low polyclonal production |
| 27-15 | low | Low suppression |
| 15-5 | medium | Medium suppression |
| <5 | high | High suppression |

Alternatively total FLC production can be split into three bands, high production, normal or suppression production.

The ratio of κ light chain type bound to a particular heavy chain class : λ light chain bound to the same heavy chain class (HLC κ:λ) ratio may compared to a normal range for that heavy chain class. The heavy chain class may be IgA, IgM, IgD or IgE. More typically IgG, IgA or IgM as these are more likely to be associated with such diseases. One or more of the classes may be assayed. Hence the ratios may be IgA : IgAλ, IgGκ : IgGλ, IgMκ : IgMλ, IgDκ : IgDλ and/or IgEκ : IgEλ.

Typical normal ratios are:

IgGκ : IgGλ    0.98-2.75

IgAκ : IgAλ    0.80-2.04

IgMκ : IgMλ    0.96-2.30

These HLC ratios may be scored in a similar manner to FLC.

Where the HLCκ : HLCλ ratio is within the normal range, the total HLC for that class (HLCκ + HLCλ) may be determined and scored to give an indication of elevation or suppression of heavy chain class production. That is, for example, IgGκ : IgGλ (the same class) is then determined.

Where the FLCκ : FLCλ and the HLCκ : HLCλ are within the normal range, elevated levels of FLCκ or FLCλ and/or HLCκ or HLCλ may indicated disease and highlight that further investigations are required. Elevation may be defined by levels above the normal range. Typical normal ranges are:

FLCκ 3.3-19.4

FLCλ 5.71-26.3

IgGκ 4.03- 9.78

IgGλ 1.97- 5.71

IgAκ 0.48- 2.82

IgAλ 0.36- 1.98

IgMκ 0.29- 1.82

IgMλ 0.17- 0.94

Instead of or additionally the HLCκ : HLCλ ratio may be subclass specific. This gives more specific data. IgG for example has four subclasses (IgG1, IgG2, IgG3 and IgG4). IgA has two subclasses (IgA1 and IgA2). Hence the HLC ratio may be, for example, IgG2κ : IgG2λ ratio.

Examples of typical scoring systems are:

Table 4

| Heavy Chain Class | HLCκ : HLCλ | Score | Result |
|---|---|---|---|
| G | >30 | high | IgGκ high |
| | 10-30 | medium | IgGκ medium |
| | 2.75-10 | low | IgGκ low |
| | 0.98-0.75 | low | IgGλ low |
| | 0.75-0.3 | medium | IgGλ medium |
| | <0.3 | high | IgGλ high |
| A | >60 | high | IgAκ high |
| | 15-60 | medium | IgAκ medium |
| | 2.04-15 | low | IgAκ low |
| | 0.8-0.7 | low | IgAλ low |
| | 0.7-0.5 | medium | IgAλ medium |
| | <0.5 | high | IgAλ high |
| M | >30 | high | IgMκ high |
| | 10-30 | medium | IgMκ medium |
| | 2.30-10 | low | IgMκ low |
| | 0.96-0.7 | low | IgMλ low |
| | 0.7-0.2 | medium | IgMλ medium |
| | <0.2 | high | IgMλ high |

Polyclonality can be similarly scored.

Table 5

| Heavy Chain Class | Total HLC (g/L serum) | Score | Result |
|---|---|---|---|
| G | >26 | high | High polyclonal IgG |
| | 19-26 | medium | Medium polyclonal IgG |
| | 9-19 | low | Low polyclonal IgG |
| | 9-6 | low | Low suppressed IgG |
| | 6-3 | medium | Medium suppressed IgG |
| | <3 | high | High suppressed IgG |
| A | >16 | high | High polyclonal IgA |
| | 5-16 | medium | Medium polyclonal IgA |
| | 2-5 2 (normal) | low | Low polyclonal IgA |
| | 2.0-0.8 | low | Low suppressed IgA |
| | 0.8-0.3 | medium | Medium suppressed IgA |
| | <0.3 | high | High suppressed IgA |
| M | >7 | high | High polyclonal IgM |
| | 3-7 | medium | Medium polyclonal IgM |
| | 1.5-3 3 (normal) | low | Low polyclonal IgM |
| | 1.5-0.4 | low | Low suppressed IgM |
| | 0.4-0.2 | medium | Medium suppressed IgM |

(continued)

| Heavy Chain Class | Total HLC (g/L serum) | Score | Result |
|---|---|---|---|
| | <0.2 | high | High suppressed IgM |

Again, a score of "zero" may be given for a normal ratio. Suppression and production can also be indicated as three bands, high production, normal and suppressed.

Clonality may also be scored in a similar manner to FLC ratios for IgG and indeed similarly for other heavy chains.

Table 6

| Heavy Chain Class | Ratio | Amount of HLC (g/L) | Amount of Other HLC (g/L) | Score |
|---|---|---|---|---|
| G | Abnormal | IgG κ > 9.78 | IgG λ < 1.97 | +++IgG κ |
| | | | 1.97 < IgG < λ 5.71 | ++IgG κ |
| | | | IgG λ > 5.71 | +IgG |
| | | IgG λ > 5.71 | IgG κ < 4.03 | +++IgG λ |
| | | | 4.03 < IgG κ < 9.78 | ++IgG λ |
| | Normal (0.98 - 2.75) | IgG κ + IgG λ > 15.49 | | Production |
| | | IgG κ + IgGλ < 6 | | Suppression |
| A | Abnormal | IgA κ > 2.82 | IgA λ < 0.36 | +++IgA κ |
| | | | 0.36 < IgA λ < 1.98 | ++IgA κ |
| | | | IgA λ > 1.98 | +IgA |
| | | IgA λ > 1.98 | IgA κ < 0.48 | +++IgA λ |
| | | | 0.48 < IgA κ < 2.82 | ++IgA λ |
| | Normal (0.8 - 2.04) | IgA κ + IgA λ > 4.8 | | Production |
| | | IgA κ + IgA λ < 0.84 | | Suppression |
| M | Abnormal | IgM κ > 1.82 | IgM λ < 0.17 | +++IgM κ |
| | | | 0.17 < IgM λ < 0.94 | ++IgM κ |
| | | | IgM λ > 0.94 | +IgM |
| | | IgM λ > 0.94 | IgM κ < 0.29 | +++IgM λ |
| | | | 0.29 < IgM κ < 1.82 | ++IgM λ |
| | Normal (0.96 - 2.30) | IgM κ + IgM λ > 2.74 | | Production |
| | | IgM κ + IgM λ < 0.46 | | Suppression |

For example a moderate IgAλ with some polyclonal M suppression and analytical error on the IgGκ (low weighting and disagreement) could be expressed as IgGκ low, IgAλ medium, IgM total medium suppression.

The FLC results (κ:λ FLC and/or total FLC) can be compared with the HLC results (HLCκ:HLCλ and/or total HLC) to identify errors or areas of agreement.

An IgA lambda with high λ FLC, polyclonally suppressed IgM and analytical error on IgG would be expressed as:

κ:λ FLC λ high, IgAλ high, IgGλ low, IgM total low suppression.

The alternative ways of scoring described above can be used as follows.

For example, do the FLC (β) results indicate clonality kappa [A] or lambda [B] or a polyclonal abnormality [C]. [A] or [B] can be scored on a level of 1 to 4 (+,++, +++ or ++++). [C] can be suppression or elevation and so be scored on a measure of -4 to +4 (---, --, -, 0, +, ++, +++ or ++++). This means that the FLC result will be one of the following:

$$A\left.\right\}^{+4}_{+1}, \quad B\left.\right\}^{+4}_{+1}, \quad C\left.\right\}^{+4}_{-4}$$

For example a strong FLC lambda result the FLC measure ($\beta$) would be:

$$B\left.\right\}^{+4}$$

The HLC results for G ($\gamma$), A ($\alpha$) or M ($\mu$) can be depicted in the same manner, i.e. do they indicate clonality (kappa [A] or lambda [B] or a polyclonal abnormality [C]. Once again, clonality [A] or [B] can be scored on a level of 1 to 4 (+, ++, +++ or ++++) and for [C] suppression or elevation can occur and so be scored on a measure of -4 to +4 (-----, ---, --, -, 0, +, ++, +++, ++++).

$$A\left.\right\}^{+4}_{+1}, \quad B\left.\right\}^{+4}_{+1}, \quad C\left.\right\}^{+4}_{-4}$$

For example an moderate IgA lambda with some polyclonal M suppression and analytical error on the IgG kappa (low weighting and disagreement) could be expressed as follows.

$$\gamma\left[A\left.\right\}_{+1}\right], \quad \alpha\left[B\left.\right\}^{+2}\right], \quad \mu\left[C\left.\right\}_{-2}\right]$$

The FLC result ($\beta$) can then be combined with the HLC G ($\gamma$), A ($\alpha$) and M ($\mu$) results. The example of an IgA lambda with high lambda FLC levels, a polyclonally suppressed IgM results and an analytical error on the IgG would be expressed as follows:

$$\beta\left[B\left.\right\}^{+4}\right], \quad \gamma\left[A\left.\right\}_{+1}\right], \quad \alpha\left[B\left.\right\}^{+2}\right], \quad \mu\left[C\left.\right\}_{-2}\right]$$

The clonal identity is shown by the agreement between the FLC ($\beta$) result and the HLC ($\gamma$, $\alpha$ or $\mu$) result, in this case $\beta$ & $\alpha$. $\beta$ and $\alpha$ shows that this is a lambda sample, the disagreement of $\gamma$ shows that the $\gamma$ result is an error and the $\mu$ result provides the additional information that there is polyclonal suppression, that there is polyclonal suppression.
The second outcome from the algorithm is the degree of confidence in the result and this can be calculated from the items of agreement:

$$\beta\left[B\left.\right\}^{+4}\right], \quad \alpha\left[B\left.\right\}^{+2}\right]_{or} \left.\right\}^{+2}_{} + \left.\right\}^{+4} = 8$$

The measurements of the FLC and HLC in the sample(s) may be determined using methods generally known in the art.
Antibodies, or fragments of antibodies, specific for $\kappa$ or $\lambda$ FLC are generally know and are commercially available under the trade name Freelite™.

Heavy chain class - light chain type - specific antibodies (for example anti-IgAκ antibodies) are also generally known the art (see WO 2006/079816) and are commercially available under the trade name Hevylite™ from The Binding Site, Birmingham, UK. Alternatively the light chains bound to heavy chains can be determined via, for example, a sandwich assay using anti-light chain type antibodies and anti-heavy chain class antibodies as described in WO 2006/079816.

Typically the FLC or HLC, such as κ:λ FLC ratio, total FLC, HLCκ:HLCλ and/or total HLC is determined by immunoassay, such as ELISA assays or utilising fluorescently labeled beads, such as Luminix™ beads.

ELISA, for example uses antibodies to detect specific antigens. One or more of the antibodies used in the assay may be labeled with an enzyme capable of converting a substrate into a detectable analyte. Such enzymes include horseradish peroxidase, alkaline phosphatase and other enzymes known in the art. Alternatively other detectable tags or labels may be used instead of, or together with, the enzymes. These include radioisotopes, a wide range of coloured and fluorescent labels known in the art including fluorescein, Alexa fluor, Oregon Green, BODIPY, rhodamine red, Cascade Blue, Marina Blue, Pacific Blue, Cascade Yellow, gold; and conjugates such as biotin (available from, for example, Invitrogen Ltd, United Kingdom). Dye sols, metallic sols, chemiluminescent labels or coloured latex may also be used. one or more of these labels may be used in the ELISA assays according to the various inventions described therein, or alternatively in the other assays, labeled antibodies or kits described herein.

The construction of ELISA-type assays is itself well known in the art. For example, a "binding antibody" specific for the FLC is immobilised on a substrate. The "binding antibody" may be immobilised onto the substrate by methods which are well known in the art. FLC in the sample are bound by the "binding antibody" which binds the FLC or HLC to the substrate via the "binding antibody".

Unbound immunoglobulins may be washed away.

In ELISA assays the presence of bound immunoglobulins may be determined by using a labeled "detecting antibody" specific to a different part of the FLC of interest than the binding antibody.

Flow cytometry may be used to detect the binding of the FLC or HLC of interest. This technique is well known in the art for, for example, cell sorting. However, it can also be used to detect labeled particles, such as beads and to measure their size. Numerous text books describe flow cytometry, such as Practical Flow Cytometry, 3rd Ed. (1994), H. Shapiro, Alan R. Liss, New York, and Flow Cytometry, First Principles (2nd Ed.) 2001, A.L. Given, Wiley Liss.

One of the binding antibodies, such as the antibody specific for FLC, is bound to a bead, such as a polystyrene or latex bead the beads are mixed with the sample and the second detecting antibody. The detecting antibody is preferably labeled with a detectable label, which binds the FLC to be detected in the sample. This results in a labeled bead when the FLC to be assayed is present.

Other antibodies specific for other analytes described herein may also be used to allow the detection of those analytes.

Labeled beads may then be detected via flow cytometry. Different labels, such as different fluorescent labels may be used for, for example, the anti-free λ and anti-free κ antibodies. Other antibodies specific for other analytes described herein may also be used in this or other assays described herein to allow the detection of those analytes. This allows the amount of each type of FLC bound to be determined simultaneously or the presence of other analytes to be determined.

Alternatively, or additionally, different sized beads may be used for different antibodies, for example for different marker specific antibodies. Flow cytometry can distinguish between different sized beads and hence can rapidly determine the amount of each FLC or other analyte in a sample.

An alternative method uses the antibodies bound to, for example, fluorescently labeled beads such as commercially available Luminex ™ beads. Different beads are used with different antibodies. Different beads are labeled with different fluorophore mixtures, thus allowing different analytes to be determined by the fluorescent wavelength. Luminex beads are available from Luminex Corporation, Austin, Texas, United States of America.

Preferably the assay used is a nephelometric or turbidimetric method.

Apparatus configured to carry out the method of the invention are also provided. They may comprise a computer processor and memory configured to carry out the method. They may comprise an output, such as display screen, to display the score(s) and/or result.

The apparatus may comprise a detector for measuring the κ:λ FLC ratio, HLCκ:HLCλ ratios and optionally the total FLC and/or total HLC.

The apparatus may be a flow cytometer, nephelometer or turbidometer.

The invention also provides the use in a method according to claims 1 to 13 of a kit comprising, in combination, (i) anti-κ FLC specific and anti-λ FLC specific antibodies or fragments thereof and (ii) anti- κ heavy chain class specific and anti- λ heavy chain class specific antibodies or fragments thereof, optionally mixed together, preferably additionally comprising further antibodies or fragments thereof for determining the total FLC is a sample.

Such detecting antibodies or fragments may be mixed together, for example, in a multiplex kit, using different dyes or other labels for each different detecting antibody. The labels may, for example, be different Luminex™ beads.

The application may be described by may of example only.

κ : λ FLC ratios, total FLC, HLCκ : HLCλ ratios and/or total HLC may be carried out using techniques generally known in the art.

Typically Freelite™ kits, Hevylite™ kits and Combylite™ kits (available from The Binding Site Ltd, Birmingham, UK) are used according to manufacturers instructions to measure samples of serum from patients.

Results are scored as, for example, shown in Tables 1 to 6 above, and as described above.

Table 7 below shows the interpretation of some of the results that might be expected, based on the types of patients found previously by the applicants.

Table 7

| FLC outcome | HLC outcome | | | Interpretation |
|---|---|---|---|---|
| | HLC IgG | HLC IgA | HLC IgM | |
| FLCλ-MEDIUM | IgGλ-MEDIUM | NORMAL IgA RATIO - MEDIUM SUPPRESSION | IgMλ-MEDIUM | IgGλ, IgMλ, IgMλ, FLCλ |
| FLCλ-HIGH | NORMAL IgG RATIO - HIGH SUPPRESSION | NORMAL IgA RATIO - HIGH SUPPRESSION | IgMλ-MEDIUM | FLCλ, IgMλ |
| FLCκ-HIGH | NORMAL IgG RATIO - MEDIUM SUPPRESSION | NORMAL IgA RATIO - HIGH SUPPRESSION | IgMκ-LOW | FLCκ, -IgMκ |
| NORMAL FLC RATIO - MEDIUM SUPPRESSION | NORMAL IgG RATIO - MEDIUM SUPPRESSION | IgAλ-LOW | NORMAL IgM RATIO - LOW SUPPRESSION | IgAλ |
| NORMAL FLC RATIO - HIGH SUPPRESSION | IgGλ-LOW | IgAλ-MEDIUM | NORMAL IgM RATIO - HIGH SUPPRESSION | IgAλ |
| FLOCκ-LOW | NORMAL IgG RATIO - MEDIUM SUPPRESSION | NORMAL IgA RATIO - LOW SUPPRESSION | NORMAL IgM RATIO - LOW SUPPRESSION | FLCκ |
| NORMAL FLC RATIO - LOW SUPPRESSION | NORMAL IgG RATIO - MEDIUM SUPPRESSION | NORMAL IgA RATIO - LOW SUPPRESSION | NORMAL IgM RATIO - HIGH SUPPRESSION | non-clonal, with systemic polyclonal suppresson |
| FLCλ-LOW | NORMAL IgG RATIO - LOW SUPPRESSION | NORMAL IgA RATIO - LOW SUPPRESSION | NORMAL IgM RATIO-MEDIUM SUPPRESSION | FLCλ |
| FLCλ-LOW | NORMAL IgG RATIO - LOW SUPPRESSION | NORMAL IgA RATIO - LOW SUPPRESSION | NORMAL IgM RATIO - HIGH SUPPRESSION | FLCκ |
| NORMAL FLC RATIO - LOW SUPPRESSION | NORMAL IgG RATIO - LOW POLYCLONAL PRODUCTION | NORMAL IgA RATIO - LOW SUPPRESSION | NORMAL IgM RATIO - HIGH SUPPRESSION | non-clonal |
| NORMAL FLC RATIO LOW SUPPRESSION | NORMAL IgG RATIO - HIGH SUPPRESSION | IgAλ-MEDIUM | IgMλ-LOW | IgAλ |
| NORMAL FLC RATIO - LOW POLYCLONAL PRODUCTION | NORMAL IgG RATIO - LOW POLYCLONAL PRODUCTION | NORMAL IgA RATIO - LOW SUPPRESSION | IgMλ-LOW | IgMλ |

(continued)

| FLC outcome | HLC outcome | | | Interpretation |
|---|---|---|---|---|
| | HLC IgG | HLC IgA | HLC IgM | |
| NORMAL FLC RATIO - LOW POLYCLONAL PRODUCTION | NORMAL IgG RATIO - LOW POLYCLONAL PRODUCTION | NORMAL IgA RATIO - LOW POLYCLONAL PRODUCTION | NORMAL IgM RATIO - LOW SUPPRESSION | **non-clonal** |
| NORMAL FLC RATIO - MEDIUM POLYCLONAL PRODUCTION | NORMAL IgG RATIO - LOW POLYCLONAL PRODUCTION | NORMAL IgA RATIO - LOW POLYCLONAL PRODUCTION | IgMκ-LOW | **IgMκ** |
| NORMAL FLC RATIO - MEDIUM SUPPRESSION | NORMAL IgG RATIO - MEDIUM SUPPRESSION | NORMAL IgA RATIO - MEDIUM SUPPRESSION | NORMAL IgM RATIO - HIGH SUPPRESSION | **non-clonal,** with systemic **polyclonal suppresson** |
| FLOCκ-LOW | NORMAL IgG RATIO - MEDIUM SUPPRESSION | NORMAL IgA RATIO - LOW SUPPRESSION | NORMAL IgM RATIO - LOW SUPPRESSION | **FLCκ** |

Non-clonal suggests that further investigation may be necessary.

Demonstration of the use of an Algorithm of the Invention

**Methods:** 1515 patients referred to the Royal Wolverhampton Hospital were analysed. SPE and IFE were performed using a SEBIA Hydrasys 2, FLC and HLC assays were performed nephelometrically, and the results were analysed using an algorithm as described herein that incorporated:

(1) FLC and HLC ratios:

(2) the levels of isotype-match suppression;

(3) the levels of systemic immunoglobulin suppression; and

(4) FLC and HLC production.

The results from the algorithm and the historic SPE and IFE gels were compared with patient's clinical diagnosis.
**Results:** At presentation, 156/1515 (10%) samples had an abnormal SPE; 101/156 which were positive by IFE. 52/101 of the IFE positive patients had confirmed haematological disorders, included:

14 multiple myeloma (MM),
3 Waldenström macroglobulinemia (WM),
1 IgM cryoglobulinemia,
3 chronic lymphocytic leukaemias (CLL),
3 lymphoma,
1 plasmacytoma
1 Sjogren's syndrome, and
26 monoclonal gammopathy with undetermined significance (MGUS).

At presentation, 175/1515 (11%) samples were identified by the FLC/HLC algorithm as having FLC and/or HLC abnormalities. The FLC/HLC algorithm identified 78 patients with haematological disorders, including all 52 patients identified by SPE/IFE, and a further 26 patients not detected by SPE/IFE, included:

1 amyloidosis,
1 asymptomatic MM,

6 CLL,
1 small lymphocytic leukaemia
2 lymphoma, and
15 light chain MGUS patients.

**Conclusion:** An algorithm based on FLC and HLC assays provides a more sensitive method for detecting haematological disorders and identified 26 patients missed by a screening panel of SPE and sIFE.

**Claims**

1. A method for characterising a plasma cell associated disease in a patient comprising:

   (i) providing at least one sample from the patient;
   (ii) determining in the sample(s) two or more of;

      (a) the $\kappa:\lambda$ free light chain (FLC) ratio;
      (b) the ratio of $\kappa$ light chains bound to a class of heavy chain : $\lambda$ light chain bound to the same class of heavy chain (HLC $\kappa$ : HLC $\lambda$ ratio);
      (c) the total amount of FLC in the samples and
      (d) the total amount of $\kappa$ light chains bound to the heavy chain class plus $\lambda$ light chains bound to the same heavy chain class (total HLC);

   (iii) comparing each ratio or amount from (a) (b), (c) and/or (d) to predetermined values and assigning a score to each amount or ratio; and
   (iv) using the scores to characterise the plasma cell associated disease.

2. A method according to claim 1, wherein the FLC $\kappa:\lambda$ ratio and HLC $\kappa:\lambda$ ratio are determined.

3. A method according to claim 1 or 2 wherein each of (a), (b), (c) and (d) are determined.

4. A method according to claim 1 to 3, wherein the amount of $\kappa$ FLC, $\lambda$ FLC, $\kappa$ HLC or $\lambda$ HLC is determined, in combination with one or more of (a), (b), (c) or (d).

5. A computer-implemented method comprising the method of claims 1 to 4.

6. A method according to claims 1 to 4, wherein step (iii) is implemented by computer.

7. A method according to any preceding claim, wherein the scores indicate the type of plasma cell disease and/or indicate a confidence level for the characterisation of the plasma cell disease.

8. A method according to any preceding claims, wherein the $\kappa:\lambda$ free light chain ratio is determined and compared to a normal range for FLC $\kappa:\lambda$, preferablywherein if the FLC $\kappa:\lambda$ ratio is above or below the normal range the FLC $\kappa:\lambda$ is given a score, more preferably wherein if the FLC $\kappa:\lambda$ is within the normal range, the total amount of FLC in the sample is determined and a score is given for the amount, and even more preferably wherein if the FLC$\kappa$ : $\lambda$ ratio is within the normal range, a FLC$\kappa$ or FLC$\lambda$ level above the normal range indicates that further investigation is required.

9. A method according to any preceding claim, wherein HLC$\kappa$:HLC$\lambda$ ratio is determined and compared to a normal range, the HLC$\kappa$:HLC$\lambda$ for that heavy chain class, preferably wherein if the HLC$\kappa$:HLC$\lambda$ ratio is above or below the normal range, the HLC$\kappa$:HLC$\lambda$ ratio is given a score, more preferably wherein if the HLC$\kappa$:HLC$\lambda$ ratio is within the normal range, the total HLC$\kappa$ + HLC$\lambda$ amount is determined and given a score, even more preferably wherein if the HLC$\kappa$ : HLC$\lambda$ ratio is within the normal range, a HLC$\kappa$ or HLC$\lambda$ level above the normal range indicates further investigation is required.

10. A method according to any preceding claim, wherein the HLC$\kappa$:HLC$\lambda$ ratio and/or total HLC is subclass specific.

11. A method according to any preceding claim wherein the amount of $\lambda$ FLC in the sample is compared to the amount of the $\kappa$ FLC to produce a clonality score or an indication of production or suppression of the FLCs.

**12.** A method according to any preceding claim wherein the amount of HLC $\lambda$ in the sample is compared to the amount of HLC $\kappa$ to produce a clonality score or an indication of the production or suppression of the FLC.

**13.** A method according to any preceding claim, wherein the $\kappa$:$\lambda$ FLC ratio, HLC$\kappa$:HLC$\lambda$ ratio, total FLC and/or total HLC is determined by an immunosorbent assay, preferably comprising the use of anti-$\kappa$ FLC-specific, anti-$\lambda$ FLC-specific, anti-$\kappa$ heavy chain class-specific and/or anti-$\lambda$ heavy chain class-specific antibodies, or specific fragments thereof, more preferably wherein the assay is a sandwich assay, such as an ELISA assay, or the binding of antibodies to the FLC or HLC is determined using a nephelometer, a turbidometer, flow cytometer and/or Luminex™ beads.

**14.** Apparatus comprising a computer processor and memory configured to carry out a method according to any preceding claim, comprising a detector for measuring the $\kappa$:$\lambda$ FLC ratio, HLC$\kappa$:HLC$\lambda$ ratio and optionally the total FLC and/or total HLC.

**15.** Use in a method according to claims 1 to 13 of a kit comprising, in combination, (i) anti-$\kappa$ FLC specific and anti-$\lambda$ FLC specific antibodies or fragments thereof and (ii) anti- $\kappa$ heavy chain class specific and anti- $\lambda$ heavy chain class specific antibodies or fragments thereof, optionally mixed together, preferably additionally comprising further antibodies or fragments thereof for determining the total FLC in a sample.

**Patentansprüche**

**1.** Verfahren zum Charakterisieren einer Plasmazellen-assoziierten Erkrankung bei einem Patienten, umfassend:

(i) Bereitstellen mindestens einer Probe des Patienten;
(ii) in der Probe beziehungsweise den Proben, Bestimmen von zwei oder mehr von:

(a) dem Verhältnis von $\kappa$:$\lambda$ der freien Leichtketten (FLC);
(b) dem Verhältnis von an eine Schwerkettenklasse gebundenen $\kappa$-Leichtketten : an dieselbe Schwerkettenklasse gebundenen $\lambda$-Leichtketten (Verhältnis HLC $\kappa$ : HLC$\lambda$);
(c) der gesamten Menge der FLC in den Proben und
(d) der gesamten Menge der an die Schwerkettenklasse gebundenen $\kappa$-Leichtketten plus der an dieselbe Schwerkettenklasse gebundenen $\lambda$-Leichtketten (Gesamt-HLC);

(iii) Vergleichen eines jeden Verhältnisses oder einer jeden Menge aus (a), (b), (c) und/oder (d) mit vorbestimmten Werten und Zuweisen eines Scores zu jeder Menge oderjedem Verhältnis und
(iv) Verwenden der Scores, um die Plasmazellen-assoziierte Erkrankung zu charakterisieren.

**2.** Verfahren gemäß Anspruch 1, bei dem das FLC-$\kappa$:$\lambda$-Verhältnis und das HLC-$\kappa$:$\lambda$-Verhältnis bestimmt werden.

**3.** Verfahren gemäß Anspruch 1 oder 2, bei dem ein jedes von (a), (b), (c) und (d) bestimmt wird.

**4.** Verfahren gemäß Anspruch 1 bis 3, bei dem die Menge von $\kappa$-FLC, $\lambda$-FLC, $\kappa$-HLC oder $\lambda$-HLC in Kombination mit einem oder mehreren von (a), (b), (c) oder (d) bestimmt wird.

**5.** Computerimplementiertes Verfahren, welches das Verfahren der Ansprüche 1 bis 4 umfasst.

**6.** Verfahren gemäß den Ansprüchen 1 bis 4, bei dem Schritt (iii) durch Computer implementiert ist.

**7.** Verfahren gemäß einem der vorstehenden Ansprüche, bei dem die Scores den Typ der Plasmazellerkrankung anzeigen und/oder ein Konfidenzniveau für die Charakterisierung der Plasmazellerkrankung anzeigen.

**8.** Verfahren gemäß einem der vorstehenden Ansprüche, bei dem das Verhältnis von $\kappa$:$\lambda$ der freien Leichtketten bestimmt wird und mit einem Normalbereich für FLC $\kappa$:$\lambda$ verglichen wird, wobei bevorzugt, wenn das FLC-$\kappa$:$\lambda$-Verhältnis über oder unter dem Normalbereich liegt, das FLC $\kappa$:$\lambda$ einen Score erhält, wobei weiter bevorzugt, wenn das FLC $\kappa$:$\lambda$ im Normalbereich liegt, die gesamte Menge der FLC in der Probe bestimmt wird und ein Score für die Menge erteilt wird, und wobei noch weiter bevorzugt, wenn das Verhältnis FLC$\kappa$ : $\lambda$ im Normalbereich liegt, ein FLC$\kappa$- oder FLC$\lambda$-Niveau über dem Normalbereich anzeigt, dass eine weitere Untersuchung erforderlich ist.

9. Verfahren gemäß einem der vorstehenden Ansprüche, bei dem das Verhältnis HLCκ:HLCλ bestimmt wird und mit einem Normalbereich, dem HLCκ:HLCλ für diese Schwerkettenklasse, verglichen wird, wobei bevorzugt, wenn das Verhältnis HLCκ:HLCλ über oder unter dem Normalbereich liegt, das Verhältnis HLCκ:HLCλ einen Score erhält, wobei weiter bevorzugt, wenn das Verhältnis HLCκ:HLCλ im Normalbereich liegt, die gesamte Menge HLCκ + HLCλ bestimmt wird und einen Score erhält, wobei noch weiter bevorzugt, wenn das Verhältnis HLCκ:HLCλ im Normalbereich liegt, ein HLCκ- oder HLCλ-Niveau über dem Normalbereich anzeigt, dass eine weitere Untersuchung erforderlich ist.

10. Verfahren gemäß einem der vorstehenden Ansprüche, bei dem das Verhältnis HLCκ:HLCλ und/oder die Gesamt-HLC subklassenspezifisch sind.

11. Verfahren gemäß einem der vorstehenden Ansprüche, bei dem die Menge von λ-FLC in der Probe mit der Menge der κ-FLC verglichen wird, um einen Klonalitäts-Score oder ein Anzeichen für die Erzeugung oder Unterdrückung der FLCs zu erzeugen.

12. Verfahren gemäß einem der vorstehenden Ansprüche, bei dem die Menge der HLC λ in der Probe mit der Menge der HLC κ verglichen wird, um einen Klonalitäts-Score oder ein Anzeichen für die Erzeugung oder Unterdrückung der FLC zu erzeugen.

13. Verfahren gemäß einem der vorstehenden Ansprüche, bei dem das Verhältnis κ:λ-FLC, das Verhältnis HLCκ:HLCλ, die Gesamt-FLC und/oderdie Gesamt-HLC durch einen Immunsorptionsassay bestimmt werden, der bevorzugt die Verwendung von Anti-κ-FLC-spezifischen, Anti-λ-FLC-spezifischen, Anti-κ-Schwerkettenklassen-spezifischen und/oder Anti-λ-Schwerkettenklassen-spezifischen Antikörpern oder spezifischen Fragmenten derselben umfasst, wobei der Assay weiter bevorzugt ein Sandwich-Assay wie etwa ein ELISA-Assay ist oder die Bindung von Antikörpern an die FLC oder HLC mit einem Nephelometer, einem Turbidimeter, Flusszytometer und/oder Luminex™-Beads bestimmt wird.

14. Vorrichtung, die einen Computerprozessor und Speicher umfasst, welche zum Durchführen eines Verfahrens gemäß einem der vorstehenden Ansprüche ausgebildet sind, umfassend einen Detektor zum Messen des Verhältnisses κ:λ-FLC, des Verhältnisses HLCκ:HLCλ und optional der Gesamt-FLC und/oder der Gesamt-HLC.

15. Verwendung, in einem Verfahren gemäß den Ansprüchen 1 bis 13, eines Kits, das in Kombination (i) Anti-κ-FLC-spezifische und Anti-λ-FLC-spezifische Antikörper oder Fragmente derselben und (ii) Anti-κ-Schwerkettenklassen-spezifische und Anti-λ-Schwerkettenklassen-spezifische Antikörper oder Fragmente derselben, optional miteinander vermischt, umfasst und bevorzugt zusätzlich weitere Antikörper oder Fragmente derselben zum Bestimmen der Gesamt-FLC in einer Probe umfasst.

**Revendications**

1. Procédé permettant de caractériser une maladie associée aux cellules du plasma chez un patient comprenant des étapes consistant à :

   (i) se procurer au moins un échantillon du patient,
   (ii) déterminer dans l'échantillon au moins deux valeurs parmi les valeurs suivantes :

      (a) le rapport chaînes légères (FLC) κ libres : chaînes légères (FLC) λ libres,
      (b) le rapport chaînes légères κ liées à une classe de chaînes lourdes : chaînes légères λ liée à la même classe de chaînes lourdes (rapport HLC κ : HLC λ),
      (c) la quantité totale de FLC dans l'échantillon, et
      (d) la quantité totale de chaînes légères κ liées à la classe de chaînes lourdes et de chaînes légères λ liée à la même classe de chaînes lourdes (HLC total),

   (iii) comparer chaque rapport ou quantité de (a), (b), (c) et/ou (d) à des valeurs prédéfinies, et attribuer un score à chacune de ces quantités ou à chacun de ces rapports, et
   (iv) utiliser les scores pour caractériser la maladie associée aux cellules du plasma.

2. Procédé conforme à la revendication 1 selon lequel le rapport FLC κ : FLC λ et le rapport HLC κ : HLC λ sont déterminés.

3. Procédé conforme à la revendication 1 ou 2 selon lequel chacune des valeurs (a), (b), (c) et (d) est déterminée.

4. Procédé conforme à la revendication 1 à 3 selon lequel la quantité de FLC κ, FLC λ, HLC κ ou HLC λ est déterminée en combinaison avec l'une ou plusieurs des valeurs (a), (b), (c) et (d).

5. Procédé assisté par ordinateur comprenant le procédé conforme à l'une des revendications 1 à 4.

6. Procédé conforme à l'une des revendications 1 à 4 selon lequel l'étape (iii) est assistée par ordinateur.

7. Procédé conforme à l'une quelconque des revendications précédentes selon lequel les scores indiquent le type de maladie des cellules du plasma et/ou indiquent un niveau de certitude pour la caractérisation de la maladie des cellules du plasma.

8. Procédé conforme à l'une quelconque des revendications précédentes selon lequel le rapport chaînes légères κ libres : chaînes légères λ libres est déterminé et comparé à une plage normale du rapport FLC κ : FLC λ, et de préférence si le rapport FLC κ : FLC λ est supérieur ou inférieur à la plage normale, un score est attribué au rapport FLC κ : FLC λ, de façon plus préférentielle, si le rapport FLC κ : FLC λ est situé dans la plage normale, la quantité totale de FLC dans l'échantillon est déterminée et un score est attribué à cette quantité, et de façon encore plus préférentielle, si le rapport FLC κ : FLC λ est situé dans la plage normale, un niveau de FLC κ ou de FLC λ situé au-dessus de la plage normale indique que des recherches complémentaires sont nécessaires.

9. Procédé conforme à l'une quelconque des revendications précédentes selon lequel le rapport HLC κ : HLC λ est déterminé et comparé à une plage normale du rapport HLC κ : HLC λ pour cette classe de chaînes lourdes, de préférence, si le rapport HLC κ : HLC λ est supérieur ou inférieur à la plage normale, un score est attribué au rapport HLC κ : HLC λ, de façon plus préférentielle, si le rapport HLC κ : HLC λ est situé dans la plage normale, la quantité totale HLC κ + HLC λ est déterminée et un score lui est attribué, et, de façon encore plus préférentielle, si le rapport HLC κ : HLC λ est situé dans la plage normale, un niveau de HLC κ ou de HLC λ situé au-dessus de la plage normale indique que des recherches complémentaires sont nécessaires.

10. Procédé conforme à l'une quelconque des revendications précédentes selon lequel le rapport HLC κ : HLC λ et/ou la quantité totale de HLC est spécifique à une sous-classe.

11. Procédé conforme à l'une quelconque des revendications précédentes selon lequel la quantité de FLC λ dans l'échantillon est comparée à la quantité de FLC κ pour obtenir un score de clonalité ou une indication de production ou de suppression des FLC.

12. Procédé conforme à l'une quelconque des revendications précédentes selon lequel la quantité de HLC λ dans l'échantillon est comparée à la quantité de HLC κ pour obtenir un score de clonalité ou une indication de la production ou de la suppression de FLC.

13. Procédé conforme à l'une quelconque des revendications précédentes selon lequel le rapport FLC κ : FLC λ, le rapport HLC κ : HLC λ, le FLC total et/ou le HLC total est déterminé par un essai d'immuno-absorption, comprenant de préférence l'utilisation d'anticorps anti FLC κ spécifiques, d'anticorps anti FLC λ spécifiques, d'anticorps anti classe de chaînes lourdes κ spécifiques et/ou d'anticorps anti classe de chaînes lourdes λ spécifiques, ou de fragments spécifiques de ces anticorps, et de façon plus préférentielle, l'essai est un essai en sandwich tel qu'un essai ELISA ou la liaison d'anticorps aux FLC ou aux HLC est déterminée en utilisant un néphélomètre, un turbidomètre, un cytomètre de flux et/ou des perles Luminex™.

14. Appareil comprenant un processeur de calcul et une mémoire conformé pour mettre en oeuvre un procédé conforme à l'une quelconque des revendications précédentes comprenant un détecteur pour permettre de mesurer le rapport FLC κ : FLC λ, le rapport HLC κ : HLC λ et le cas échéant, la quantité de FLC totale et/ou la quantité de HLC totale.

15. Utilisation dans un procédé conforme à l'une des revendications 1 à 13 d'un kit comprenant en combinaison :

   (i) des anticorps anti FLC κ spécifiques et des anticorps anti FLC λ spécifiques ou des fragments de tels anticorps, et

(ii) des anticorps anti classe de chaînes lourdes κ spécifiques et des anticorps anti classe de chaînes lourdes λ spécifiques ou des fragments de tels anticorps, le cas échéant mélangés, et de préférence comprenant en outre d'autres anticorps ou fragments de tels anticorps pour permettre de déterminer la quantité de FLC totale dans un échantillon.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011021041 A
- WO 2006079816 A

### Non-patent literature cited in the description

- **A.R. BRADWELL.** Serum Free Light Chain Analysis. 2008
- **KATZMANN et al.** *Clin. Chem.,* 2002, vol. 48 (9), 1437-1944
- **DISPENZIERI A.** *Leukaemia,* 2009, vol. 23-2, 215-24
- **H. SHAPIRO.** Practical Flow Cytometry. Alan R. Liss, 1994
- **A.L. GIVEN.** Flow Cytometry. Wiley Liss, 2001